# EUROPÄISCHE PATENTSCHRIFT

(11) **EP 1 295 117 B1**
(45) Veröffentlichungstag und Bekanntmachung des Hinweises auf die Patenterteilung: **20.01.2010**
(21) Anmeldenummer: 01925472.1
(22) Anmeldetag: 15.03.2001
(51) Int. Cl.: G01N 33/00, G01N 27/62, G01N 27/64, G01M 3/20

(54) **TESTGASDETEKTOR FÜR EIN LECKSUCHGERÄT MIT EINEM SENSOR FÜR HELIUM ODER WASSERSTOFF**
TESTGAS DETECTOR FOR A LEAK DETECTION DEVICE COMPRISING A SENSOR FOR HELIUM OR HYDROGEN
DETECTEUR DE GAZ-TEST POUR LA DETECTION DE FUITE CONTENANT UN CAPTEUR DE L'HELIUM OU DE L'HYDROGENE

(30) Priorität: 30.06.2000 DE 10031882
(43) Veröffentlichungstag der Anmeldung: 26.03.2003
(73) Patentinhaber: Inficon GmbH, 50968 Köln (DE)
(72) Erfinder: GROSSE BLEY, Werner, 53125 Bonn (DE)
(74) Vertreter: Leineweber, Jürgen
(86) Internationale Anmeldenummer: PCT/EP2001/002970
(87) Internationale Veröffentlichungsnummer: WO 2002/003057

(56) Entgegenhaltungen:
- EP-A- 0 352 371
- DE-A- 4 326 265
- DE-A- 4 326 267
- DE-A- 19 521 275
- FR-A- 1 310 649
- US-A- 3 051 868
- US-A- 3 280 619
- US-A- 3 411 073
- US-A- 4 477 778

## Beschreibung

Die Erfindung bezieht sich auf einen Testgasdetektor für ein Lecksuchgerät mit den Merkmalen des Oberbegriffs des Patentanspruchs 1.

Ein Testgasdetektor mit diesen Merkmalen ist aus der DE 195 21 275 A1 bekannt. Ein Testgasdetektor ähnlicher Art, bei dem der Gaseinlass als Quarzglasscheibe oder als Polymermembran ausgebildet ist, ist aus der DE-A 43 26 265 A1 bekannt.

Testgasdetektoren der bekannten Art weisen einen relativ einfachen Aufbau auf und können bis annähernd Atmosphärendruck (außerhalb ihres Gehäuses) betrieben werden. Dennoch konnten sich Sensoren dieser Art bisher nicht im Markt durchsetzen, insbesondere weil sie im Vergleich zu den üblicherweise verwendeten, allerdings teureren Massenspektrometern zu unempfindlich sind und deshalb nicht unter Vakuumbedingungen mit geringen Prüfgaspartialdrücken eingesetzt werden können.

Der vorliegenden Erfindung liegt die Aufgabe zugrunde, einen Testgasdetektor der Eingangs erwähnten Art mit verbesserten Eigenschaften zu schaffen. Erfindungsgemäß wird diese Aufgabe durch die kennzeichnenden Merkmale der Patentansprüche gelöst.

Dadurch, dass das Gehäuse aus Glas besteht, findet keine H₂-Gas Abgabe innerhalb des Gehäuses statt. Vorzugsweise wird Borosilikatglas verwendet, da es sich sowohl zum Einschmelzen entsprechend thermisch angepaßter Metalle eignet als auch für das anodische Bonden bzw. Diffusionsschweißen mit Silizium geeignet ist. Der beim Sensor nach der Erfindung verwendete Gasdurchlass (an sich bekannt aus der DE-A-195 21 275) erlaubt den Aufbau eines relativ großflächigen Gasdurchlasses mit ausreichender Stabilität. Schließlich hat die polymer- und elastomerfreie Verbindung des Gasdurchlasses mit dem Gehäuse durch anodisches Bonden oder Diffusionsschweißen (an sich bekannt aus der FR 1310 649 A1) den Vorteil, dass eine Gasabgabe aus Dichtungen oder Klebstoffen in das Innere des Gehäuses nicht stattfindet. Alle diese Maßnahmen wirken sich vorteilhaft auf die Empfindlichkeit des Sensors aus bzw. ergeben eine Verbesserung der Nachweisgrenze.

Weitere Vorteile und Einzelheiten der Erfindung sollen anhand von in den Figuren 1 bis 4 schematisch dargestellten Ausführungsbeispielen erläutert werden. Es zeigen
- Figuren 1 bis 3 eine Ausführungsform mit einem im wesentlichen quaderförmigen Gehäuse und
- Figur 4 eine Ausführungsform mit Magnetrongeometrie.

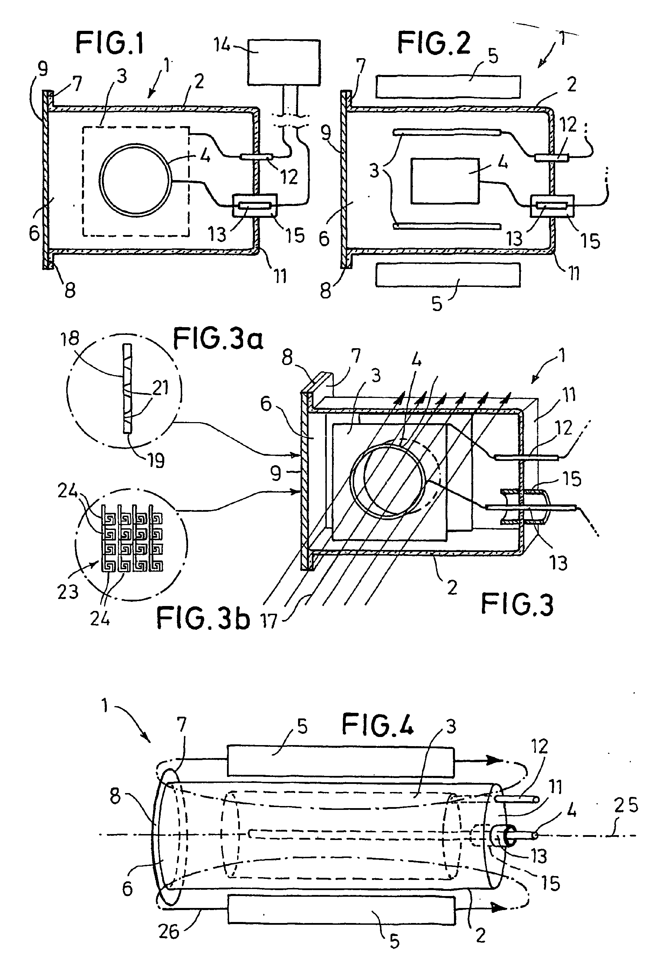

In allen Figuren sind das Gehäuse des Sensors 1 mit 2, die sich innerhalb des Gehäuses 2 befindenden Katodenbauteile mit 3 sowie Anodenbauteile mit 4 und die außerhalb des Gehäuses 2 angeordneten Magneten mit 5 bezeichnet. Das im übrigen geschlossen ausgebildete Gehäuse 2 weist jeweils eine offene Stirnseite 6 auf. In diesem Bereich ist es mit einem flanschartigen Rand 7 ausgerüstet, dessen stirnseitige Ringfläche 8 der Herstellung der Verbindung des Gasdurchlasses 9 (nur in den Figuren 1 bis 3 dargestellt) mit dem Gehäuse 2 durch anodisches Bonden oder Diffusionsschweißen dient. Die der offenen Stirnseite 6 bzw. dem Gasdurchlaß 9 gegenüberliegende Stirnseite 11 des Gehäuses 2 ist mit elektrischen Durchführungen 12 und 13 versehen, die über Leitungen mit einem lediglich als Block 14 dargestellten Steuer-, Meß-, Registrier-, Anzeige- und/oder Versorgungsgerät in Verbindung stehen. Durchführung 12 dient der Versorgung der Katodenbauteile 3 mit Hochspannung. Durchführung 13 verbindet die Anode 4 mit dem Gerät 14. Diese Durchführung 13 ist mit einer Abschirmung 15 ausgerüstet, da die durchgeführte Leitung der Zuführung extrem kleiner Ionenströme (bis in den fA-Bereich) zum Gerät 14 dient. Als Werkstoff für die Durchführungen 12, 13 und für die Abschirmung 15 haben sich Eisen/(Kobalt)/Nickel-Legierungen (Handelsnamen VACON, KOVAR,...) am zweckmäßigsten erwiesen. Wolfram und Platin sind mit besonderen Glassorten u.U. auch einsetzbar.

Bei den in den Figuren 1 bis 3 dargestellten Ausführungen hat das Gehäuse 2 im wesentlichen die Form eines Quaders. Innerhalb des Gehäuses 2 befinden eine Ringanode aus Edelstahl 4 und zwei etwa quadratische, vorzugsweise aus Titanblechabschnitten bestehende Kathoden 3, die den Stirnseiten der Ringanode 3 in der üblichen Weise vorgelagert sind. Die Elektroden sind zur Minimierung von Wasserstoffabgabe in einem Entgasungsprozeß unter Hochvakuum bei ca. 600°-900°C geglüht worden.

Figur 3 ist eine perspektivische Darstellung. Die dem Betrachter zugewandte Gehäusewand fehlt. Das von den Magneten 5, vorzugsweise Permanentmagneten, erzeugte Magnetfeld ist durch Pfeile 17 gekennzeichnet.
Figur 3 umfaßt zwei Ausschnittvergrößerungen 3a und 3b des Gasdurchlasses 9. Figur 3a zeigt einen Schnitt durch den Gasdurchlaß 9. Er besteht im wesentlichen aus einer Membran 18 mit den gewünschten selektiven Eigenschaften und aus einem Träger 19. Der Träger 19 ist eine Siliziumscheibe, in die mit Hilfe bekannter Ätzverfahren mit einer Vielzahl von Durchbrechungen 21 versehen ist. Die Durchbrechungen 21 bilden durch die Membran 18 verschlossene Fenster mit einer Fensterfläche von beispielsweise 0,5 mm². Die Summe der Fensterflächen bildet den eigentlichen Gasdurchlaß (Gesamtdurchtrittsfläche).

Der Gasdurchlaß 9 kann beispielsweise nach einem Verfahren hergestellt sein, wie es aus der DE-195 21 257 A1 bekannt ist. Dieses gilt auch für die Heizung 23, mit der der Gasdurchlaß 9 zur Erhöhung der Ansprechzeit ausrüstet ist. Figur 3b zeigt eine Ausführungsform, bei der jede der Fensterflächen mit einer aussenliegenden Heizwendel aus Nickel/Chrom oder vorzugsweise Platin 24 ausgerüstet ist. Die elektrische Versorgung der Heizwendeln erfolgt vom äusseren Rand der Struktur, wo auf jeder Schmalseite jeweils ein Pol aller Heizwendeln zusammengeführt ist.

Bei der Ausführung nach Figur 5 sind das Gehäuse 2 und die Katode 3 zylindrisch ausgebildet. Die Anode 4 hat die Form eines Stabes, der in der gemeinsamen Achse 25 von Gehäuse 2 und Katode 3 angeordnet ist. Die vom Magneten 5 erzeugten Feldlinien sind durch Pfeile 26 gekennzeichnet.

Durch die Maßnahmen nach der Erfindung soll erreicht werden, dass keinerlei Gasabgabe in das Gehäuse 2 stattfindet, um eine mit Massenspektrometern vergleichbare Partialdruckempfindlichkeit zu erzielen. Da es erforderlich ist, das z. B. aus Borosilikatglas bestehende Gehäuse 2 mit anderen Werkstoffen zu paaren bzw. dicht zu verbinden, ist es zweckmäßig, dass unterschiedliche Bereiche das Gehäuse 2 aus unterschiedlichen Glassorten mit unterschiedlichen Ausdehnungskoeffizienten bestehen. Dadurch ergibt sich die Möglichkeit, den Ausdehnungskoeffizienten des Gehäuses dort, wo es mit einem anderen Werkstoff dicht verbunden werden muß, dem Ausdehnungskoeffizienten des anderen Werkstoffes anzupassen. Die Dichtheit des Gehäuses 2 wird dadurch verbessert, Materialspannungen werden reduziert.

Zur Anpassung an Silizium mit einem thermischen Ausdehnungskoeffizienten von 3,0 ppm/K eignet sich besonders gut DURAN-Glas mit 3,3 ppm/K. Zur Anpassung an KOVAR oder VACON ist das SCHOTT Einschmelzglas 8250 mit 5,0 ppm/K optimal. Daneben ist die Paarung Platin mit Thermometerglas möglich und im Prinzip kann Wolfram mit DURAN gepaart werden. Zur Überbrückung der unterschiedlichen Ausdehnungskoeffizienten werden eine Reihe von sogenannten Übergangsgläsern angeboten, die in entsprechender Folge eingesetzt werden müssen, wenn temperaturbedingte Spannungen und damit Undichtigkeiten bis hin zur Zerstörung vermieden werden sollen.

Bei den dargestellten Ausführungsformen ist beispielsweise die im Bereich der Stirnseite 6 bzw. der anodischen Bondfläche 8 für das Gehäuse 2 verwendete Borosilikatglassorte dem Ausdehnungskoeffizienten der Siliziumscheibe 19 angepaßt, die die selektive Membran 18 trägt. Im Bereich der gegenüberliegenden Stirnseite 11 hat die dort für das Gehäuse 2 verwendete Borosilikatglassorte zweckmäßig einen Ausdehnungskoeffizienten der den dort durchgeführten Metallen (z.B. KOVAR oder VACON) entspricht. Im Übergangsbereich zwischen den Stirnseiten 6 und 11 wird zweckmäßig eine Übergangsglassorte mit einem Ausdehnungskoeffizienten verwendet, der zwischen den beiden Ausdehnungskoeffizienten der stirnseitig verwendeten Glassorten liegt. Wenn nötig können zur Vermeidung von Werkstoffspannungen auch mehrere Übergangsglassorten mit sich in gleichsinnigen Schritten verändernden Ausdehnungskoeffizienten verwendet werden. Es ergeben sich damit folgende Fertigungsalternativen:
- Gehäuse aus DURAN-Glas (SCHOTT), Stromdurchführungen aus KOVAR oder VACON eingeschmolzen in SCHOTT 8250-Glas und mit Domen aus Übergangsgläsern an DURAN angepaßt, Siliziumscheibe direkt anodisch gebondet;
- Gehäuse aus Einschmelzglas (SCHOTT 8250), Stromdurchführungen aus KOVAR oder VACON, Siliziumscheibe mit Aluminiumzwischenlage diffusionsgeschweisst;
- Gehäuse aus DURAN-Glas (SCHOTT), Stromdurchführungen aus Wolfram, Siliziumscheibe direkt anodisch gebondet;
- Gehäuse aus DURAN-Glas (SCHOTT), Stromdurchführungen aus Platin eingeschmolzen in Thermometerglas und mit Domen aus Übergangsgläsern an DURAN angepaßt, Siliziumscheibe direkt anodisch gebondet.

Die genannten Glassorten sind Beispiele, entscheidend ist die Anpassung der jeweiligen Ausdehnungskoeffizienten von Metall und Glas.

Die Vorteile des Sensors nach der Erfindung liegen insbesondere darin, dass sie bis annähernd Atmosphärendruck betrieben werden können und ausreichend empfindlich sind. Sie können deshalb an Stelle von Massenspektrometern bei der Lecksuche verwendet werden, d. h., sie können Bestandteil des Testgasdetektors von Lecksuchgeräten sein, unabhängig davon, ob ein Prüfling an das Lecksuchgerät angeschlossen wird oder ein Schnüffler vorgesehen ist, mit dem ein Prüfling abgetastet wird.

Auch der Einsatz in Vakuumanlagen, z. B. mit sich ständig ändernden Drücken, ist vorteilhaft, und zwar sowohl als Bestandteil eines Testgasdetektors einer Lecksucheinrichtung als auch als Gassensor allgemein. Bei der Lecksuche an einer Vakuumkammer wird diese von außen mit Testgas besprüht. Im Falle eines Lecks dringt Testgas ein und wird vom Sensor registriert.

## Patentansprüche

1. Testgasdetektor für ein Lecksuchgerät mit einem Sensor (1) für Helium oder Wasserstoff; der Sensor (1) besitzt ein vakuumdichtes Gehäuse (2), in dem sich ein Vakuummessgerät (3, 4) befindet, sowie einen selektiv wirkenden Durchlass (9) für das festzustellende Gas, wobei Bestandteile des Gasdurchlasses (9) eine aus einem Siliziumwerkstoff bestehende Membran (18) mit den gewünschten selektiven Eigenschaften, eine die Membran (18) stützende, mit einer Vielzahl von Durchbrechungen (21) versehende Siliziumscheibe (19) sowie eine Heizung (24) sind, **dadurch gekennzeichnet, dass** das Gehäuse (2) aus Glas besteht, daß Bestandteil des Vakuummessgerätes eine gasaufzehrende Kaltkatodenanordnung (3, 4) ist und dass das Gehäuse (2) und der selektiv wirkende Gasdurchlass (9) durch Bonden oder Diffusionsschweißen hochvakuumdicht sowie polymer- und elastomerfrei miteinander verbunden sind.

2. Testgasdetektor nach Anspruch 1, **dadurch gekennzeichnet, dass** die Katode der Kaltkatodenanordnung aus Titanblech besteht.

3. Testgasdetektor nach Anspruch 1 oder 2, **dadurch gekennzeichnet, dass** das Gehäuse (2) aus Borosilikatglas besteht.

4. Testgasdetektor nach einem der Ansprüche 1 bis 3, **dadurch gekennzeichnet, dass** die dem Gasdurchlass abgewandte Seite des Gehäuses mit Spannungs- bzw. Stromdurchführungen ausgerüstet ist.

## Claims

1. Test gas detector for a leakage detection device comprising a sensor (1) for helium or hydrogen; the sensor (1) has a vacuum-tight housing (2), in which there is a vacuum measurement device (3, 4), and a selectively acting inlet (9) for the gas to be detected, components of the gas inlet (9) being a membrane (18) which consists of a silicon material and has the desired selective properties, a silicon wafer which supports the membrane (18) and is provided with a multiplicity of openings (21), and a heater (24), **characterised in that** the housing (2) consists of glass, **in that** one component of the vacuum measurement device is a gas-consuming cold cathode arrangement (3, 4), and **in that** the housing (2) and the selectively acting gas inlet (9) are connected to one another by bonding or by diffusion welding, in such a way as to seal against a high vacuum and be free from polymers and elastomers.

2. Test gas detector according to Claim 1, **characterised in that** the cathode of the cold cathode arrangement consists of sheet titanium.

3. Test gas detector according to one of Claims 1 or 2, **characterised in that** the housing (2) consists of borosilicate glass.

4. Test gas detector according to one of Claims 1 to 3, **characterised in that** the opposite side of the housing from the gas inlet is equipped with feed-throughs for voltage and/or current.

## Revendications

1. Détecteur de gaz témoin pour un détecteur de fuites, comportant un capteur (1) pour l'hélium ou l'hydrogène ; le capteur (1) comporte un boîtier (2) sous vide, dans lequel est disposé un manomètre à vide (3, 4), ainsi qu'un passage (9) à action sélective pour le gaz à détecter, les parties intégrantes du passage de gaz (9) étant une membrane (18), réalisée dans un matériau de silicium et possédant les propriétés sélectives souhaitées, un disque de silicium (19), supportant la membrane (18) et muni d'une pluralité de trous débouchants (21), ainsi qu'un chauffage (24), **caractérisé en ce que** le boîtier (2) est réalisé en verre, **en ce que** la partie intégrante du manomètre à vide est un système à cathode froide (3, 4) absorbant le gaz et **en ce que** le boîtier (2) et le passage de gaz (9) à action sélective sont reliés entre eux, de manière étanche au vide très poussé et sans polymère et élastomère, par métallisation ou soudage par diffusion.

2. Détecteur de gaz témoin selon la revendication 1, **caractérisé en ce que** la cathode du système à cathode froide est réalisée dans une tôle de titane.

3. Détecteur de gaz témoin selon la revendication 1 ou 2, **caractérisé en ce que** le boîtier (2) est réalisé en verre au borosilicate.

4. Détecteur de gaz témoin selon l'une quelconque des revendications 1 à 3, **caractérisé en ce que** le côté du boîtier, opposé au passage de gaz, est muni de douilles de traversée pour la tension ou le courant.
